# FASCICULE DE BREVET EUROPEEN

(11) **EP 2 210 083 B1**
(45) Date de publication et mention de la délivrance du brevet: **16.09.2015**
(21) Numéro de dépôt: 08853924.2
(22) Date de dépôt: 13.11.2008
(51) Int. Cl.: G01N 21/64, G01N 33/84

(54) **PROCÉDÉ DE MESURE DE LA CAPACITÉ D'UN ÉCHANTILLON A RÉSISTER AUX ESPÈCES RÉACTIVES DE L'OXYGÈNE (EROs)**
VERFAHREN ZUR MESSUNG DER RESISTENZ EINER PROBE GEGEN REAKTIVE SAUERSTOFFSPEZIES
METHOD FOR MEASURING THE ABILITY OF A SAMPLE TO WITHSTAND REACTIVE OXYGEN SPECIES (ROSS)

(30) Priorité: 13.11.2007 FR 0707938; 16.04.2008 FR 0802110
(43) Date de publication de la demande: 28.07.2010
(73) Titulaire: Patrice, Thierry, 44800 Saint-Herblain (FR)
(72) Inventeur: Patrice, Thierry, 44800 Saint-Herblain (FR)
(74) Mandataire: Godineau, Valérie
(86) Numéro de dépôt international: PCT/FR2008/052042
(87) Numéro de publication internationale: WO 2009/068820

(56) Documents cités:
- WO-A-92/10759
- WO-A-2004/034058
- BILSKI P ET AL: "Photosensitized oxidation of 2',7'-dichlorofluorescin: singlet oxygen does not contribute to the formation of fluorescent oxidation product 2',7'-dichlorofluorescein." FREE RADICAL BIOLOGY & MEDICINE 1 OCT 2002, vol. 33, no. 7, 1 octobre 2002 (2002-10-01), pages 938-946, XP002500240 ISSN: 0891-5849
- VARGAS FRANKLIN R ET AL: "Antioxidant and scavenging activity of emodin, aloe-emodin, and rhein on free-radical and reactive oxygen species" PHARMACEUTICAL BIOLOGY, vol. 42, no. 4-5, juin 2004 (2004-06), pages 342-348, XP009107274

## Description

La présente invention concerne un procédé de mesure de la capacité, dite capacité anti-oxydante totale (TAS), d'un échantillon biologique issu d'un organisme humain ou animal à résister aux espèces réactives de l'oxygène (EROs).

Elle concerne plus particulièrement un procédé utilisant une réaction photochimique pour la mesure de la résistance d'un liquide biologique, d'un tissu ou d'un extrait tissulaire et plus généralement d'un matériau issu d'un organisme humain ou animal vivant ou non au stress oxydant et aux espèces réactives oxydantes (EROs) produites lors du métabolisme normal ou pathologique ou induites lors de la mise en oeuvre d'un traitement.

Le stress oxydant a fait l'objet de nombreuses études ces dernières années en raison de son implication dans un grand nombre de processus normaux, pathologiques ou thérapeutiques. Des tests permettant de quantifier tel ou tel facteur impliqué dans la défense vis-à-vis des EROs ont été élaborés. Des systèmes permettant la mesure de la capacité antioxydante totale (TAS) ont également été développés : Imanox TAS kit, Randox Kit (EP-0.563.114), WO 03/016527. L'invention se situe dans le cadre de la mesure du TAS. Par ailleurs l'identification de sources potentielles d'EROs ont été étudiées et parmi elles il a été montré que les réactions photochimiques produisent des EROs dont la nature et la quantité varient en fonction de la nature chimique du photosensibilisant, de l'intensité de la lumière délivrée au photosensibilisant et de la nature du tissu qui a été sensibilisé par le photosensibilisant préalablement à l'irradiation lumineuse. Dans ce contexte, l'ERO primaire le plus réactif formé en plus grande quantité est l'oxygène singulet (¹O₂).

Le stress oxydant et les EROs sont produits lors de l'exposition de matériaux à des agents chimiques ou physiques, par exemple de la matière plastique exposée au soleil, ou en biologie dans les réactions physiologiques de phagocytose par les globules blancs aboutissant à la destruction de bactéries par exemple, mais aussi et surtout lors de réactions biochimiques nombreuses du métabolisme normal ou pathologique. Les EROs produites en excès ou insuffisamment neutralisées vont avoir des effets délétères pouvant conduire au vieillissement accéléré de constituants biologiques ou non, à la dégradation précoce de tissus biologiques ou même de matériaux. Parmi les pathologies directement ou indirectement liées aux EROs, on peut citer la maladie d'Alzheimer, le diabète et ses conséquences, certains cancers, certaines pathologies dégénératives du tissu osseux et du cartilage, l'athérosclérose. Plus généralement une hypothèse du vieillissement des êtres vivants repose aussi sur une action délétère oxydative des EROs qui altèrent de manière diffuse l'ensemble des constituants biologiques protéiques, lipidiques et/ou glucidiques.

Parmi les EROs on peut citer 0₂.- radical superoxyde, HO₂. radical perhydroxyle, .OH radical hydroxyle, RO₂. radical peroxyle, RO. radical alkoxyle, .NO radical nitroxyl. Ces EROs sont des radicaux libres, c'est-à-dire une espèce chimique possédant un électron célibataire sur sa couche périphérique. Ces radicaux libres qui interviennent ont en effet un électron célibataire sur un atome d'oxygène. ¹O₂ ou oxygène singulet est quant à lui, une des deux formes excitées de l'oxygène. Cet oxygène singulet est non radicalaire et dépourvu d'électron célibataire.

La durée de vie de l'oxygène singulet est de quelques quelques microsecondes en milieu aqueux.

L'oxygène singulet est produit soit par une décharge entre electrodes, soit chimiquement, soit par reaction photochimique, le rose bengal ayant un rendement quantique de production de ¹0₂ très élevé de l'ordre de 0,75. L'oxygène singulet va réagir avec différents substrats pour former des espèces réactives de l'oxygène (EROs primaires) qui vont eux même se désactiver progressivement en formant des EROs secondaires de plus faible potentiel oxydant et ainsi de suite en fonction du temps.

Les radicaux libres vont altérer diverses cibles biologiques en particulier les lipides, protéines et acides nucléiques. Ces espèces radicalaires sont impliquées dans de nombreuses pathologies regroupées sous le terme de stress oxydant. Les facteurs influençant en l'augmentant l'action des EROs sont nombreux au premier rang desquels la pression partielle en oxygène dans le tissu et donc le pH ainsi que d'un point de vue biologique tous les enzymes de régulation de la mort cellulaire. Les facteurs influençant en la diminuant l'action des EROs sont également nombreux : Protéines, vitamines, superoxyde dismutase (SOD), système glutathion/glutathion peroxydase etc. L'action de l'ERO va être une sorte de compromis entre sa réactivité, sa durée de vie, son affinité vis-à-vis de telle ou telle cible ou constituant biologique. Les substances antioxydantes en fait présentent une très forte affinité pour l'ERO et la détournent ainsi d'un autre constituant qui aurait autrement été attaqué par l'ERO. Malgré cela le composé, y compris une éventuelle substance antioxydante, subissant l'attaque de l'ERO va à son tour devenir oxydant et potentiellement délétère, quoique avec une réactivité moindre que l'ERO. Tout réactif ajouté, y compris pour la détection et à fortiori pour provoquer ou inhiber la réaction oxydante, peut d'ailleurs, dans certaines circonstances, se comporter de la même manière en présence d'EROs. Ainsi la détoxification des EROs est-elle une sorte de cascade d'événements aboutissant à la neutralisation progressive de l'ERO en fonction du temps.

Pour étudier les EROs on peut analyser leur production en les détectant directement, mais ce n'est pas possible pour la plupart des espèces en particulier c'est difficile pour ¹O₂ (mesure de la durée de vie de luminescence de l'¹O₂ à 1270nm, 5 µs dans l'eau), ou indirectement et pour la plupart d'entre elles impossible à mettre en place simplement dans un tissu ou un matériau donné en particulier les tissus broyés ou liquides biologiques du fait de la décroissance rapide de la réactivité et de l'impossibilité de réaliser une mesure en temps réel de longue durée. On peut analyser la transformation d'un marqueur coloré ou fluorescent sous l'influence des EROs produits ou circulants. Mais la réaction qui aboutit à la production des EROs aboutit aussi souvent à la destruction du marqueur. Dans un test commercialisé, on fait agir une substance chimique oxydante avec le substrat dont on veut mesurer la résistance aux EROs (TAS). Dans ce cas, cependant, le réactif qui demeure en solution fait partie de la mesure puisque la substance n'est pas neutralisée et donc source d'artefact. Tel est le cas par exemple de l'article de VARGAS FRANKLIN R ET AL. : "Antioxidant and scavenging activity of emodin, aloe-emodin, and rhein on free-radical and reactive oxygen species" PHARMACEUTICAL BIOLOGY, vol 42, no 4-5, juin 2004 (2004-06), pages 342-348. Ce document décrit un procédé pour déterminer la capacité anti-oxydante de plusieurs composés organiques. Le procédé est basé sur une réaction d'oxygène sous forme de ¹O₂ avec les composés mentionnés et la détection de la concentration de ¹O₂ par fluorescence pendant cette réaction. Dans la mise en oeuvre, la réaction photochimique qui génère la production d'oxygène singulet s'opère en présence du révélateur, c'est-à-dire d'un composé colorimétrique, en l'occurrence, le luminol. Le fait de soumettre le luminol à une irradiation lumineuse génère des artefacts. De ce fait, dans ce document, le luminol détecte directement l'oxygène singulet produit par réaction photochimique.

Le document BILSKI P ET AL : "Photosensitized oxidation of 2', 7'-dichlorofluorescin : singlet oxygen does not contribute to the formation of fluorescent oxidation product 2',7'-dichlorofluorescein." FREE RADICAL BIOLOGY & MEDICINE 1 OCT 2002, vol. 33, n° 7, 1 octobre 2002 (2002-10-01), pages 938-946 mentionne dans son titre que l'oxygène singulet ne contribue pas à la formation de dichlorofluorescéine fluorescente. A nouveau, dans ses expériences avec un échantillon biologique, BILSKI procède de manière analogue à VARGAS, c'est-à-dire qu'il irradie le composant colorimétrique estimant que toute autre démarche en particulier avec un agent, tel que de la dichlorofluorescéine, aboutit à des résultats erronés ou non interprétables.

Le document WO 92/10759 ne décrit aucune réaction photochimique. Il en est de même du document WO 2004/034058.

Par ailleurs les applications à ce jour ne font pas mention de mesures globales réalisées à l'échelle d'un tissu. Enfin on peut étudier individuellement les facteurs qui influent sur la réactivité des EROs soit en les dosant, soit en mesurant leur transformation sous l'influence des EROs, ce qui est fastidieux, coûteux et ne tient pas compte des interactions entre quenchers.

C'est pour palier l'ensemble de ces difficultés et pour satisfaire au besoin d'une mesure globale de la capacité de résistance aux EROs aussi appelée TAS et correspondant à la mesure de la capacité à inhiber les EROs que le procédé objet de la présente invention a été mis au point.

Un but de la présente invention est donc de proposer un procédé de mesure dont la mise en oeuvre permet de réduire, voire de supprimer les sources d'artefact et d'obtenir une mesure fiable et reproductible.

A cet effet, l'invention a pour objet un procédé de mesure de la capacité, dite capacité anti-oxydante totale (TAS) d'un échantillon biologique issu d'un organisme humain ou animal, à résister aux espèces réactives de l'oxygène (EROs),
caractérisé en ce que ledit procédé comprend au moins les étapes suivantes :
- mettre en contact l'échantillon biologique à tester et un agent photosensible en milieu liquide pour former un mélange à tester,
- soumettre ledit mélange à tester à une dose d'irradiation lumineuse à une longueur d'onde absorbée par l'agent photosensible pour provoquer, par réaction photochimique au moins entre lumière et agent photosensible, au moins la production d'oxygène singulet apte à former avec ledit échantillon des espèces réactives de l'oxygène (EROs),
- ajouter, après irradiation, un composé qui réagit colorimétriquement en présence d'espèces réactives de l'oxygène (EROs) pour former une substance chromogène ou fluorescente,
- mesurer dans le temps la quantité de substance chromogène ou fluorescente produite en vue de déterminer la capacité dudit échantillon à résister aux espèces réactives de l'oxygène (EROs) en les inhibant, un faible niveau de production de substance chromogène ou fluorescente correspondant à une capacité de résistance élevée aux espèces réactives de l'oxygène (EROs) dudit échantillon,
- soumettre au moins un mélange de référence formé d'un mélange échantillon biologique de référence issu d'un organisme présumé sain/agent photosensible aux mêmes réactions photochimique, colorimétrique et de mesure que le mélange à tester,
- comparer les résultats de la mesure sur le mélange à tester avec les résultats de mesure obtenus sur ledit au moins un mélange de référence.

La mesure de la quantité de substance chromogène ou fluorescente produite s'opère en fonction du temps.

Grâce à l'addition du composé apte à former une substance chromogène ou fluorescente avec les EROs uniquement après irradiation lumineuse, le composé n'est pas dégradé par ladite irradiation. En outre, on ne mesure pas la réaction de la substance chromogène ou fluorescente avec l'oxygène singulet mais avec des espèces réactives de l'oxygène elles-mêmes issues de la réaction de l'oxygène singulet avec l'échantillon biologique à tester. Ainsi, le caractère éphémère de l'oxygène singulet devient un atout.

Il doit être noté qu'il peut également être utilisé le mélange (échantillon à tester et un agent photosensible) et le mélange (échantillon de référence/agent photosensible) comme témoins permettant de corriger les données obtenues par réactions photochimique et colorimétrique en soumettant lesdits témoins uniquement à une réaction colorimétrique.

Lors de la mise en oeuvre d'un procédé du type dans lequel le mélange de l'échantillon à tester et d'un agent photosensible d'une part, et le mélange d'un échantillon de référence et d'un agent photosensible d'autre part, sont soumis aux mêmes réactions photochimique, colorimétrique et de mesure, le procédé comprend, lors de l'étape de mesure, la mesure dans le temps, pour chaque mélange, de la quantité de substance chromogène ou fluorescente produite sous forme de l'intensité du signal lumineux ou fluorescent de ladite substance, cette étape de mesure étant suivie d'une étape de traitement desdites mesures, au cours de laquelle on établit pour chaque mélange, la courbe intensité du signal lumineux de la substance chromogène en fonction du temps, puis on calcule l'aire sous la courbe et on détermine le rapport des aires du mélange à tester et du mélange de référence.

Comme mentionné ci-dessus, les résultats obtenus à partir de la seule réaction colorimétrique sans réaction photochimique du mélange à tester et du mélange de référence peuvent être soustraits des courbes obtenues après réactions photochimique et colorimétrique.

De préférence, on utilise un agent photosensible produisant majoritairement l'oxygène singulet ¹O₂, ledit agent étant de préférence choisi dans le groupe formé par le rose bengale et la Tetra (4 sulfonato-phényl) porphyrine (TPPS).

De préférence encore, on choisit un agent photosensible qui, en l'absence de lumière, ne réagit pas avec l'échantillon à tester et on irradie ledit agent photosensible à une longueur d'onde choisie correspondant au maximum d'absorption du spectre de l'agent photosensible.

Généralement, avant mise en contact de l'échantillon à tester et de l'échantillon de référence avec l'agent photosensible en vue d'une irradiation lumineuse, on mesure l'absorbance de l'échantillon à tester et de l'échantillon de référence.

En effet, un point important est de mesurer l'absorbance finale de la solution dans laquelle on veut mesurer le TAS et ce avant de provoquer la réaction photochimique par l'irradiation lumineuse. En effet, la lumière servant à exciter l'agent sensible provoquant la réaction photochimique servant à mesurer le TAS va être influencée par cette absorbance et par suite l'intensité de la réaction photochimique servant à mesurer le TAS sera modifiée et notamment diminuée si l'absorbance est trop forte. De la même façon, la mesure de fluorescence émise après la réaction photochimique va être influencée par l'absorbance de la solution. La mesure de l'aire sous la courbe doit donc être calculée en fonction de l'absorbance et ce pour des valeurs d'absorbance induisant une variation linéaire des aires sous la courbe, ce qui implique un étalonnage.

En d'autres termes, une valeur d'absorbance de l'échantillon à tester représentative par exemple d'un échantillon turbide ou trop coloré permet d'exclure ledit échantillon du reste de l'analyse car il apparaît évident qu'un tel échantillon posera des problèmes en particulier car il ne laissera pas bien passer la lumière lors de la réaction photochimique et induira donc une mesure optique erronée lors de la réaction colorimétrique.

Le composé qui réagit pour former une substance chromogène est de préférence le système dichlorofluorescéine réduite-dichlorofluorescéine (DCFH-DCF).

Généralement, on met en oeuvre les réactions photochimique et colorimétrique à pH neutre ou voisin du pH biologique dans une enceinte thermostatée.

De préférence, l'échantillon biologique à tester issu d'un organisme humain ou animal est un fluide biologique, tel que du sérum, du plasma ou un extrait tissulaire en solution. L'échantillon biologique de référence est de même nature que l'échantillon biologique à tester et est issu d'un organisme porteur présumé sain. De préférence, pour chaque mélange, on mesure la quantité de substance chromogène ou fluorescente produite sous forme de l'intensité du signal lumineux ou fluorescent de ladite substance, pendant une période de temps au moins égale à 45 minutes, de préférence comprise entre 50 et 90 minutes.

L'invention sera bien comprise à la lecture de la description suivante d'exemples de réalisation, en référence aux dessins annexés dans lesquels :
la figure 1 représente, sous forme de vue schématique générale, le procédé objet de l'invention dans une version destinée à mesurer la capacité anti-oxydante totale TAS (Total Antioxydant Status) d'un matériau ou milieu biologique ;
la figure 2 représente l'évolution du signal de la fluorescence de la DCF (Dichlorofluorescéine) exprimé en unité arbitraire en fonction du temps et de la concentration en sérum de veau foetal FCS de l'échantillon à tester après irradiation 10 J/cm² en présence de rose bengale à une concentration de 10 mg/mL ;
la figure 3 représente la moyenne de l'évolution du signal de fluorescence de la DCF (Dichlorofluorescéine) exprimé en unité arbitraire en fonction du temps sur 40 analyses du même échantillon de sérum de veau foetal FCS et illustre la reproductibilité du procédé objet de la présente invention ;
la figure 4 représente l'évolution du signal de fluorescence de la DCF (Dichlorofluorescéine) exprimé en unité arbitraire en fonction du temps dans un milieu contenant du sérum et préalablement soumis à une irradiation lumineuse en présence de rose bengale dans une atmosphère normale ou saturée en argon ou en azote ;
la figure 5 représente la valeur de l'aire sous la courbe du signal de fluorescence de la DCF (Dichlorofluorescéine), en fonction de l'âge et du sexe, de sérum prélevé chez 24 hommes et 20 femmes présumés sains et soumis à une irradiation lumineuse en présence de rose bengale ;
la figure 6 représente la valeur de l'aire sous la courbe du signal de fluorescence de la DCF (Dichlorofluorescéine) de 17 échantillons de sérum prélevé chez des patients diabétiques et soumis à une irradiation lumineuse en présence de rose bengale par rapport à des sérums humains sains de référence et
la figure 7 représente la valeur de l'aire sous la courbe du signal de fluorescence de la DCF (Dichlorofluorescéine) dans du sérum prélevé chez des animaux issus de différentes espèces (nombre d'individus minimum par espèce = 8) et soumis à une irradiation lumineuse en présence de rose bengale.

Comme mentionné ci-dessus, le procédé, objet de l'invention, qui consiste à mesurer la capacité d'un échantillon biologique à résister aux espèces réactives de l'oxygène (EROs), comprend au moins les étapes successives suivantes :
a) une étape de mise en contact de l'échantillon à tester et d'un agent photosensible en milieu liquide pour former un mélange à tester,
b) une étape de soumission dudit mélange à tester à une dose d'irradiation lumineuse pour provoquer au moins la production d'oxygène singulet apte à former avec ledit échantillon des espèces réactives de l'oxygène,
c) une étape d'addition audit mélange irradié, après irradiation, d'un composé qui réagit colorimétriquement en présence d'espèces réactives de l'oxygène (EROs) pour former une substance chromogène ou fluorescente,
d) une étape de mesure de la quantité de substance chromogène ou fluorescente produite en vue de déterminer la capacité dudit échantillon à résister aux espèces réactives de l'oxygène (EROs).

Ce procédé comprend en outre la soumission d'un échantillon biologique de référence issu d'un porteur présumé sain aux étapes a, b, c, d décrites ci-dessus. La mise en oeuvre des étapes a) à d) du procédé sur les échantillons à tester et de référence peut indifféremment s'effectuer en série ou en parallèle.

Les étapes de réactions photochimique et colorimétrique et l'étape de mesure sont suivies d'une étape de comparaison des résultats. Pour réaliser cette étape de comparaison, on établit pour chaque mélange, la courbe intensité du signal lumineux de la substance chromogène en fonction du temps, puis on calcule l'aire sous la courbe et on détermine le rapport des aires du mélange à tester et du mélange de référence.

Un exemple de mise en oeuvre du procédé peut ainsi consister à ajouter au réactif photosensible, le rose bengale, présent à une concentration de 5 µg/mL, une solution à tester (par exemple sérum, FCS ou tout autre substrat consistant en un matériau biologique, tissu biologique ou liquide biologique, contenant ou non des substances dont on souhaite mesurer le potentiel antioxydant et dont on veut mesurer la capacité antioxydante en fonction du temps). Le volume des solutions d'échantillons à tester est de préférence de 1 à 50 µL. L'ensemble est en solution dans de l'eau et tamponné à pH 7,2 par ajout de tampon phosphate concentré (250mM). L'irradiation est réalisée par exemple à l'aide d'un laser émettant une fluence de 1 à 10 J/cm2 à 514 nm (de 10 à 50 secondes d'irradiation). Le révélateur, la DCFH (ou DCF réduite) est ajouté avec un dispositif automatique à la fin de l'irradiation lumineuse et agité. La concentration de ce composé est comprise entre 0,01 µg/mL et 1 mg/mL. La DCFH est donc ajoutée après que l'oxygène singulet a disparu et que des espèces réactives à l'oxygène ont commencé à être produites. Généralement, dès la fin de l'irradiation lumineuse, on ajoute de la substance chromogène. Les deux opérations sont donc sensiblement synchrones.

La fluorescence est analysée dans un système également thermostaté pour l'ensemble des solutions. Les intervalles entre les prises de mesure sont identiques pour l'ensemble des solutions. A la fin d'une période de 10 à 60 minutes, les aires sous la courbe sont calculées ainsi que les pentes permettant de caractériser la courbe d'évolution de la fluorescence à différents temps par exemple 1, 2, 5 10 20 40 et 60 minutes. Ces manipulations permettent d'obtenir des rapports d'aires sous la courbe qui vont évoluer en fonction de la concentration du sérum, de l'extrait ou autre à tester. Ainsi quand la substance à tester présentera un déficit de pouvoir antioxydant, il y aura augmentation des rapports d'aire sous la courbe et lorsque il y aura hyper protection ou activité antioxydante il y aura diminution de ces rapports par rapport aux contrôles. La normalité est appréciée par rapport à des sérums normaux ou extraits normaux de référence prélevés chez des porteurs sains.

Comme mentionné ci-dessus, la première étape du procédé a pour objet de réaliser une réaction photochimique dans des conditions parfaitement standardisées dans le but de produire, en particulier de l'oxygène singulet (¹O₂), grâce à un agent photosensible. Un agent photosensible produisant de l'oxygène singulet est placé en solution par exemple dans de l'eau. De préférence, la concentration de cet agent photosensible est comprise entre 0,01 µg/mL et 1 mg/mL. On ajoute à ladite solution un échantillon par exemple un sérum, un tissu biologique broyé ou un liquide biologique, contenant ou non des substances dont on souhaite mesurer le potentiel antioxydant (par exemple des aminothiols, Vitamine C ou E etc...) et dont on veut mesurer la capacité antioxydante sur lequel vont agir les EROs primaires et secondaires après irradiation lumineuse à une longueur d'onde absorbée par l'agent photosensible. L'ensemble est tamponné par ajout de tampon phosphate (250mM) à pH 7,2 et thermostaté. Il est procédé à une irradiation lumineuse à une longueur d'onde absorbée par l'agent photosensible. L'énergie délivrée sera telle qu'elle n'entraîne pas d'élévation thermique dans le mélange irradié qui est thermostaté. Cette irradiation lumineuse est obtenue à l'aide d'un laser ou de tout autre type de source lumineuse permettant une irradiation de longueur d'onde et d'intensité adaptées. De façon synchrone, à la fin de l'irradiation du mélange contenant l'agent photosensible et l'échantillon, on ajoute un révélateur connu ou non par exemple le système dichlorofluorescéine réduite-dichlorofluorescéine (DCFH-DCF) qui génère une fluorescence après attaque par les EROs résiduels, au fur et à mesure de la désactivation en cascade de la réactivité des EROs produites par la réaction photochimique initiale. On mesure ensuite, en fonction du temps et de manière thermostatée par cette fluorescence, l'activité oxydante des EROs résiduels.

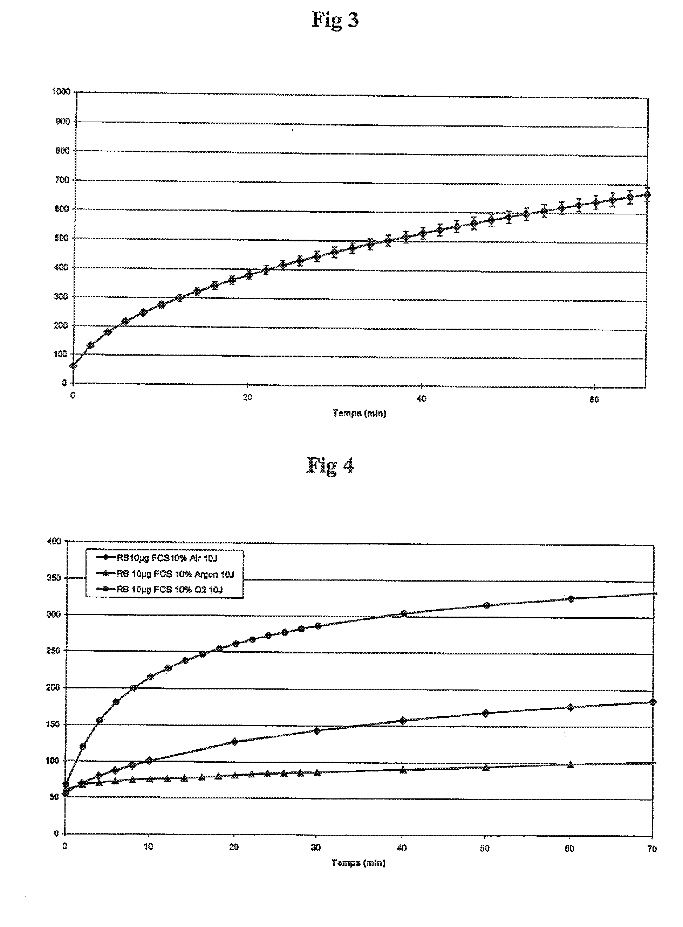

La disparition des EROs par la mesure de leur présence révélée par la DCFH-DCF est le reflet de la capacité globale en fonction du temps d'un matériau, tissu biologique ou liquide biologique à inhiber les EROs et donc à limiter leurs effets délétères au sein de l'échantillon dont on cherche à mesurer le TAS. La forme du signal obtenu grâce à un réactif coloré ou fluorescent approprié dont un exemple peut-être la DCF fluorescente après attaque de la DCFH par des EROs est caractéristique du matériau subissant la réaction photochimique et absolument différente du signal obtenu en absence du matériau ou liquide biologique. De plus, l'évolution de la fluorescence est parfaitement reproductible d'une expérience à l'autre lorsque l'irradiation lumineuse d'un sensibilisant identique est réalisée successivement 40 fois en présence d'un substrat identique (Sérum de veau foetal FCS) (Fig 3).

C'est donc bien l'évolution en fonction du temps du signal obtenu qui va être analysée et qui correspond, à la désactivation progressive des EROs produits aboutissant à une transformation de la DCFH en DCF dont la fluorescence augmente avec le nombre d'EROs qui ont interagi avec la forme non fluorescente, dite DCFH, de la DCFH-DCF. Il ne s'agit nullement d'un artefact même si on ne peut exclure une contribution de l'auto oxydation de la DCFH en DCF à ce stade. Mais, même dans ce cas, la contribution elle-même est proportionnelle à l'intensité de la production d'EROs par la réaction photochimique et inversement proportionnelle au TAS du milieu objet de la mesure. A la fin de la mesure, plus l'aire sous la courbe de la mesure de la fluorescence de la DCFH oxydée en DCF par les EROs en fonction du temps est grande et moins le matériau est capable de neutraliser rapidement les EROs primaires générés à partir de la réaction photochimique initiale standardisée. Le TAS est donc d'autant plus fort que l'aire sous la courbe est plus petite.

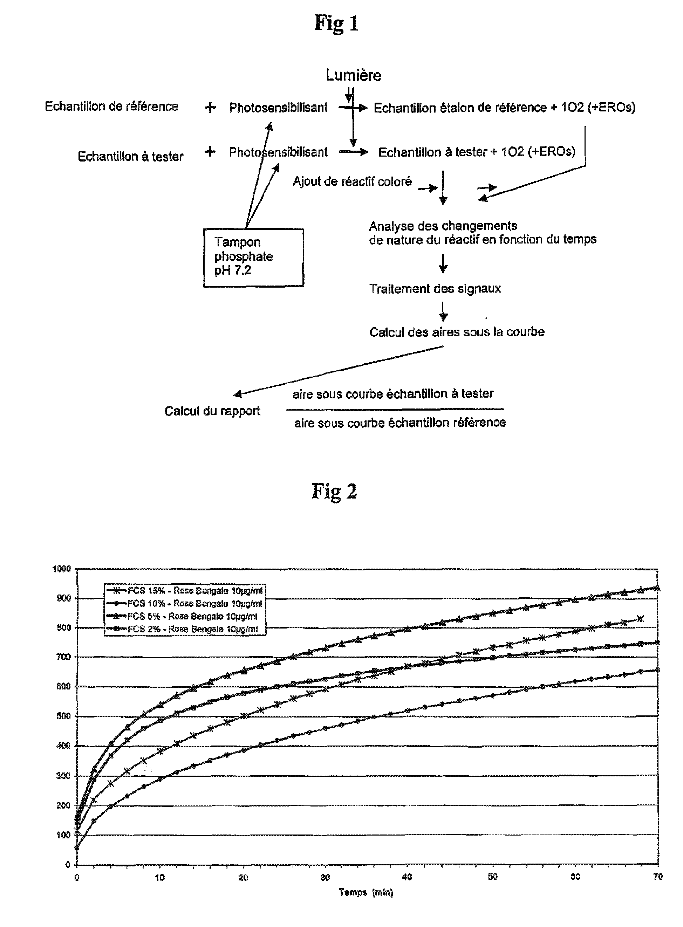

Un exemple de telles courbes est représenté à la figure 2. Chaque courbe correspond à une concentration en sérum de l'échantillon à tester. Ainsi, les échantillons présentent une concentration de 2, 5, 10 ou 15 % en sérum.

Le pH de la solution dans laquelle est effectuée l'irradiation lumineuse de l'agent photosensible permettant la production d'EROs primaires puis la détection des EROs par le système DCFH-DCF tel que décrit ci-dessus est tamponné à pH 7,2 par un tampon phosphate très concentré (250mM). Cette condition permet un bon signal de fluorescence, élimine les artéfacts liés à des variations de pH du fait des ajouts de solutés ou de composés et est compatible avec le pH biologique.

Dans une application destinée à mesurer la capacité antioxydante d'un sérum dans le but de détecter une anomalie de cette capacité antioxydante associée à une maladie, les EROs sont produites directement dans le sérum du patient à tester et comparées aux valeurs obtenues dans un sérum de référence et à des valeurs normales obtenues préalablement dans un échantillon de témoins sains.

Dans ce cas, le procédé comprend au moins une étape de soumission d'au moins un premier mélange de référence aux mêmes réactions photochimiques et colorimétriques que l'échantillon à tester. Ce premier mélange est formé par le mélange d'un agent photosensible et de sérum issu d'un panel de témoins sains. Ce procédé comprend en outre la soumission dudit mélange de référence et du mélange à tester formant un deuxième témoin à la même réaction colorimétrique que le mélange à tester sans réaction photochimique préalable. Les résultats obtenus permettent une correction des résultats.

Dans une perspective clinique, un prélèvement sanguin peut être réalisé avant traitement, immédiatement après le traitement et ensuite plus à distance. Les résultats obtenus après traitement sont comparés aux valeurs d'avant traitement. Ainsi et à titre d'exemple, il sera possible de vérifier l'impact de la Circulation extracorporelle sur le taux de complications post opératoires ou alors de mesurer l'impact de la radiothérapie sur un patient donné.

La succession d'étapes mesurant l'influence d'un tissu biologique ou liquide biologique dans la disparition des EROs va être reproduite pour des concentrations variables du tissu biologique ou liquide biologique, contenant ou non des substances dont on souhaite mesurer le potentiel antioxydant, à tester. Ainsi, les aires sous la courbe de l'évolution de la fluorescence induite par les EROs produites au sein du mélange se déplaceront en fonction de la modification du pouvoir antioxydant induite par ces matériaux, tissu biologique ou liquide biologique. Lorsque le pouvoir antioxydant augmentera, il y aura un déplacement de la courbe vers le bas. Lorsque le pouvoir antioxydant diminuera, il y aura déplacement vers le haut. Il sera ainsi possible par la mesure de la pente de la courbe de caractériser très précisément le TAS et donc autoriser les comparaisons ultérieures par exemple avec d'autres matériaux dont on voudrait mesurer le TAS.

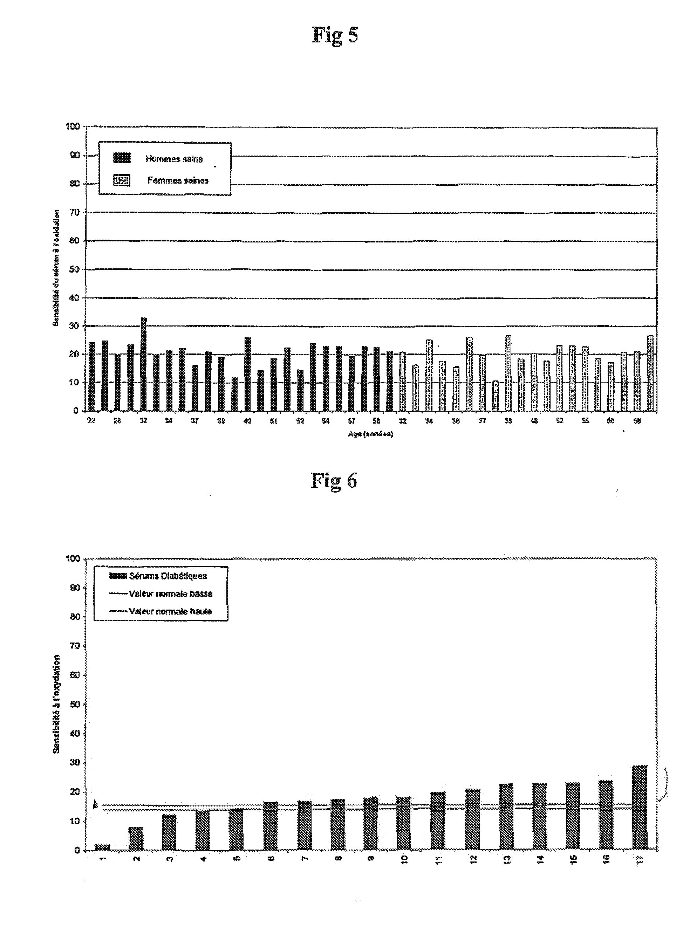

La figure 2 illustre les résultats obtenus à partir de mélange échantillon à tester + agent photosensible à différentes concentrations, un témoin formé de l'échantillon seul constituant la référence. Les résultats sont présentés en pourcentage de la valeur témoin de référence. L'aire sous la courbe est calculée pour l'échantillon à tester et pour l'échantillon de référence. Les essais réalisés en utilisant le procédé objet de l'invention et présentés ci-dessus permettent de mesurer la capacité anti oxydante de sérums humains sains (Fig 5). Il a été possible de montrer que ce potentiel était différent pour l'homme et la femme pour lesquels deux valeurs moyennes ont pu être calculées. Utilisé de la même manière sur des sérums pathologiques obtenus chez des patients atteints de diabète compliqués d'ischémie, une valeur du test supérieure aux valeurs des témoins sains a pu être mesurée (Fig 6) dans le cas de patients atteints d'un diabète instable ou compliqué. Des observations similaires ont pu être observées dans le domaine de la cancérologie avec des cancers évolutifs.

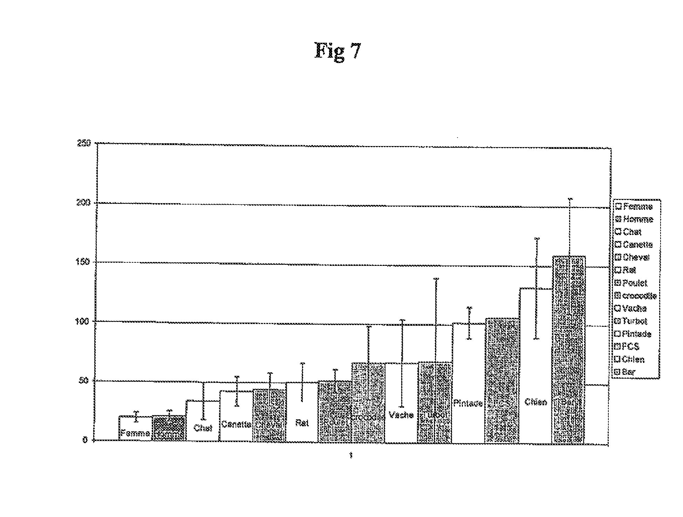

Enfin le potentiel anti oxydant a été analysé sur des sérums obtenus chez des animaux d'espèces différentes. Nous avons pu noter à la fois une variation importante entre les espèces et aussi une variation inter-individuelle importante au sein d'une espèce donnée se traduisant par un écart-type important cependant plus faible pour les sérums humains (Fig 7).

La courbe représente l'analyse de l'évolution de la fluorescence de la DCFH lors de sa transformation en DCF en fonction du temps dans les différentes solutions.

En fonction du type d'ERO vis-à-vis de laquelle on mesure la capacité à résister d'un matériau, tissu biologique ou liquide biologique, il peut être choisi un agent photosensible susceptible de produire préférentiellement tel ou tel type de ERO. Cependant il est préférable de choisir un agent photosensible produisant majoritairement l'espèce la plus énergétique c'est-à-dire ¹O₂. Ainsi un exemple de photosensibilisant utilisé pourra être le rose bengale, un autre étant la Tetra (4 sulfonato-phényl) porphyrine (TPPS), les deux ayant des rendements quantiques de production de ¹O₂ de 0,75. Un autre critère de choix est l'absence de réaction chimique entre le photosensibilisant et l'échantillon à tester lorsque le mélange est à l'abri de la lumière. L'irradiation lumineuse sera réalisée au maximum d'absorption du spectre du photosensibilisant soit pour le rose bengale (520 nm) dans le cadre d'un exemple préférentiel. L'énergie délivrée au mélange échantillon-photosensibilisant sera de 10J/cm2 dans une application préférentielle. L'irradiation lumineuse puis la détection de fluorescence devront être réalisées dans des conditions thermostatées strictes.

La figure 4 montre que la réaction photochimique initiale induit bien la production d'EROs et en particulier de ¹O₂ puisque cette réaction peut être inhibée partiellement lors d'une irradiation dans une atmosphère saturée en N₂ ou de façon encore plus nette avec une atmosphère saturée en argon (Fig 6).

Il existe différents tests permettant d'évaluer le pouvoir antioxydant de matériaux biologiques. Parmi ceux-ci la méthode de référence est appelée « Randox ». Ce procédé, objet de l'invention, diffère significativement de ce test en ce qu'il utilise une méthode optique pour produire des EROs (limitant ainsi les artefacts liés à l'introduction potentielle de contaminants par exemple métaux lourds, ou de réactifs iatrogènes). Par ailleurs l'utilisation de ¹O₂ oxygène singulet pour une telle réaction (durée de vie extrêmement brève, consommation minimale d'oxygène dans le milieu d'ou encore risque minimum d'artefact), au pouvoir oxydant maximum permet une détection globale totale des EROs induits. Par ailleurs la durée de l'irradiation étant très brève, en tout cas terminée au moment de l'ajout du réactif de détection des EROs induits immédiatement après la fin de l'irradiation lumineuse, peu importe la réactivité de ce réactif DCF à la lumière, qui ne mesure que les EROs captés. Il y a donc encore absence d'artefacts. Par ailleurs la fluorescence obtenue après réaction avec Randox est très instable le signal diminuant de 50% en 60 secondes suivant le temps de mesure dont le temps optimal préconisé par le fournisseur est de l'ordre de 3 minutes, ce qui interdit toute analyse de la disparition des EROs en fonction du temps.

La mise en oeuvre du procédé objet de l'invention peut être effectuée notamment à l'aide d'une installation se présentant sous forme d'un ensemble intégré, de préférence portable, comprenant au moins une enceinte, de préférence thermostatée, des moyens d'irradiation lumineuse de ladite enceinte, des moyens de mesure de l'intensité d'un signal lumineux à l'intérieur de ladite enceinte et des moyens d'acquisition et de traitement des données issus des moyens de mesure. Par ensemble intégré, on entend une installation comprenant l'ensemble des éléments décrits ci-dessus formant un ensemble monobloc. Les moyens d'irradiation lumineuse de l'enceinte peuvent être constitués par une simple diode. Les moyens de mesure de l'intensité d'un signal lumineux peuvent être constitués par une barrette de diodes ou de photomultiplicateurs. L'enceinte peut délimiter au moins deux compartiments, l'un des compartiments étant destiné à recevoir l'agent photosensible, l'autre l'agent apte à former une substance chromogène ou fluorescente. Lesdits compartiments sont aptes à communiquer entre eux, soit par déversement du contenu de l'un des compartiments dans l'autre, soit par rupture de la zone de liaison desdits compartiments entre eux afin de permettre, après irradiation, la réaction colorimétrique. Un troisième compartiment peut être prévu pour recevoir l'échantillon à tester et mesurer son absorbance. Grâce à une telle installation, l'opérateur se contente d'introduire l'échantillon dans l'enceinte thermostatée. Les moyens d'acquisition et/ou de traitement des données issues des moyens de mesure peuvent être également simplifiés et être réalisés essentiellement sous forme d'un traitement informatique.

Le procédé peut également être mis en oeuvre à l'aide d'une trousse de test qui comprend au moins d'une part un support délimitant au moins deux compartiments et d'autre part un agent photosensible et un agent apte à former la substance chromogène ou fluorescente disposés chacun en milieu tamponné dans l'un des compartiments du support, lesdits compartiments étant aptes à être mis en communication entre eux. A nouveau, ces compartiments peuvent être animés d'un mouvement relatif l'un par rapport à l'autre pour permettre le déversement du contenu de l'un dans le contenu de l'autre. Ces compartiments peuvent également être pourvus d'une zone de séparation amovible ou destructible qui permet, après irradiation lumineuse, la mise en communication du contenu desdits compartiments entre eux. Cette trousse de test sera destinée aux opérateurs disposant déjà de moyens d'irradiation, de moyens de mesure de l'intensité d'un signal lumineux et de moyens d'acquisition et/ou de traitement des données issues des moyens de mesure.

## Revendications

1. Procédé de mesure de la capacité, dite capacité anti-oxydante totale (TAS) d'un échantillon biologique issu d'un organisme humain ou animal, à résister aux espèces réactives de l'oxygène (EROs),
**caractérisé en ce que** ledit procédé comprenant au moins les étapes suivantes:
- mettre en contact l'échantillon biologique à tester et un agent photosensible en milieu liquide pour former un mélange à tester,
- soumettre ledit mélange à tester à une dose d'irradiation lumineuse à une longueur d'onde absorbée par l'agent photosensible pour provoquer, par réaction photochimique au moins entre lumière et agent photosensible, au moins la production d'oxygène singulet apte à former avec ledit échantillon des espèces réactives de l'oxygène (EROs),
- ajouter, après irradiation, un composé qui réagit colorimétriquement en présence d'espèces réactives de l'oxygène (EROs) pour former une substance chromogène ou fluorescente,
- mesurer dans le temps la quantité de substance chromogène ou fluorescente produite en vue de déterminer la capacité dudit échantillon à résister aux espèces réactives de l'oxygène (EROs) en les inhibant, un faible niveau de production de substance chromogène ou fluorescente correspondant à une capacité de résistance élevée aux espèces réactives de l'oxygène (EROs) dudit échantillon,
- soumettre au moins un mélange de référence formé d'un mélange échantillon biologique de référence issu d'un organisme présumé sain/agent photosensible aux mêmes réactions photochimique, colorimétrique et de mesure que le mélange à tester,
- comparer les résultats de la mesure sur le mélange à tester avec les résultats de mesure obtenus sur ledit au moins un mélange de référence.

2. Procédé selon la revendication 1,
**caractérisé en ce qu'**il comprend, lors de l'étape de mesure, la mesure dans le temps, pour chaque mélange, de la quantité de substance chromogène ou fluorescente produite sous forme de l'intensité du signal lumineux ou fluorescent de ladite substance, cette étape de mesure étant suivie d'une étape de traitement desdites mesures, au cours de laquelle on établit pour chaque mélange, la courbe intensité du signal lumineux de la substance chromogène en fonction du temps, puis on calcule l'aire sous la courbe et on détermine le rapport des aires du mélange à tester et du mélange de référence.

3. Procédé selon l'une des revendications 1 et 2,
**caractérisé en ce que**, avant mise en contact de l'échantillon à tester et de l'échantillon de référence avec l'agent photosensible en vue d'une irradiation lumineuse, on mesure l'absorbance dudit échantillon à tester et de l'échantillon de référence.

4. Procédé selon l'une des revendications 1 à 3,
**caractérisé en ce qu'**on utilise un agent photosensible produisant majoritairement l'oxygène singulet ¹O₂, ledit agent étant de préférence choisi dans le groupe formé par le rose bengale et la Tetra (4 sulfonato-phényl) porphyrine (TPPS).

5. Procédé selon l'une des revendications 1 à 4,
**caractérisé en ce qu'**on choisit un agent photosensible qui, en l'absence de lumière, ne réagit pas avec l'échantillon biologique à tester et en **en ce qu'**on irradie ledit agent photosensible à une longueur d'onde choisie correspondant au maximum d'absorption du spectre de l'agent photosensible.

6. Procédé selon l'une des revendications 1 à 5,
**caractérisé en ce que** le composé qui réagit pour former une substance chromogène est le système dichlorofluorescéine réduite-dichlorofluorescéine (DCFH-DCF).

7. Procédé selon l'une des revendications 1 à 6,
**caractérisé en ce qu'**on met en oeuvre les réactions photochimique et colorimétrique à pH neutre ou voisin du pH biologique dans une enceinte thermostatée.

8. Procédé selon l'une des revendications 1 à 7,
**caractérisé en ce que** l'échantillon biologique à tester issu d'un organisme humain ou animal est un fluide biologique, tel que du sérum, du plasma ou un extrait tissulaire en solution.

9. Procédé selon l'une des revendications 1 à 8,
**caractérisé en ce que** pour chaque mélange, on mesure la quantité de substance chromogène ou fluorescente produite sous forme de l'intensité du signal lumineux ou fluorescent de ladite substance, pendant une période de temps au moins égale à 45 minutes, de préférence comprise entre 50 et 90 minutes.

## Patentansprüche

1. Verfahren zur Messung der als antioxidative Gesamtresistenz (TAS) bezeichneten Resistenz einer biologischen Probe aus einem menschlichen oder tierischen Organismus, reaktiven Sauerstoffspezies (ROS) zu widerstehen,
**dadurch gekennzeichnet, dass** das Verfahren mindestens die folgenden Schritte umfasst:
- Inkontaktversetzen der zu testenden biologischen Probe und eines photosensiblen Mittels in wässrigem Medium, um ein zu testendes Gemisch zu bilden,
- Aussetzen des zu testenden Gemischs einer Lichtstrahlungsdosis mit einer von dem photosensiblen Mittel absorbierten Wellenlänge, um durch photochemische Reaktion mindestens zwischen Licht und photosensiblem Mittel mindestens die Produktion von Singulett-Sauerstoff hervorzurufen, der imstande ist, mit der Probe reaktive Sauerstoffspezies (ROS) zu bilden,
- Hinzufügen, nach Bestrahlung, einer Verbindung, die bei Anwesenheit reaktiver Sauerstoffspezies (POS) kolorimetrisch reagiert, um eine chromogene oder fluoreszierende Substanz zu bilden,
- Messen, im Zeitverlauf, die Menge produzierter chromogener oder fluoreszierender Substanz, um die Fähigkeit der Probe zu bestimmen, den reaktiven Sauerstoffspezies (POS) durch Hemmung zu widerstehen, wobei ein geringes Produktionsniveau chromogener oder fluoreszierender Substanz einer hohen Resistenzfähigkeit gegenüber reaktiven Sauerstoffspezies (ROS) der Probe entspricht,
- Aussetzen mindestens eines Referenzgemischs aus einem Gemisch biologische Referenzprobe eines als gesund angenommenen Organismus/photosensibles Mittel mit derselben photochemischen, kolorimetrischen und Messreaktionen wie das testende Gemisch,
- Vergleichen der Messergebnisse des zu testenden Gemischs mit den Messergebnissen des mindestens einen Referenzgemischs.

2. Verfahren nach Anspruch 1,
**dadurch gekennzeichnet, dass** es während des Messschritts für jedes Gemisch das Messen im Zeitverlauf der Menge produzierter chromogener oder fluoreszierender Substanz in Form der Intensität des Licht- oder Fluoreszenzsignals der Substanz umfasst, wobei diesem Messschritt ein Verarbeitungsschritt der Messungen folgt, bei dem für jedes Gemisch die Intensitätskurve des Lichtsignals der chromogenen Substanz in Abhängigkeit von der Zeit erstellt wird, dann der Bereich unter der Kurve berechnet und das Verhältnis der Bereiche des zu testenden Gemischs und des Referenzgemischs bestimmt wird.

3. Verfahren nach einem der Ansprüche 1 und 2,
**dadurch gekennzeichnet, dass** vor Inkontaktversetzen der zu testenden Probe und der Referenzprobe mit dem photosensiblen Mittel zwecks einer Lichtbestrahlung der Absorptionskoeffizient der zu testenden Probe und der Referenzprobe gemessen wird.

4. Verfahren nach einem der Ansprüche 1 bis 3,
**dadurch gekennzeichnet, dass** ein photosensibles Mittel verwendet wird, das mehrheitlich den Singulett-Sauerstoff ¹O₂ produziert, wobei das Mittel vorzugsweise aus der Gruppe ausgewählt ist, die von dem Bengalrosa und dem Tetra(4-sulfonatphenyl)porphyrin (TPPS) gebildet ist.

5. Verfahren nach einem der Ansprüche 1 bis 4,
**dadurch gekennzeichnet, dass** ein photosensibles Mittel gewählt wird, das bei Abwesenheit von Licht nicht mit der zu testenden biologischen Probe reagiert und dass das photosensible Mittel mit einer ausgewählten Wellenlänge bestrahlt wird, die dem Absorptionsmaximum des Spektrums des photosensiblen Mittels entspricht.

6. Verfahren nach einem der Ansprüche 1 bis 5,
**dadurch gekennzeichnet, dass** die Verbindung, die reagiert, um eine chromogene Substanz zu bilden, das System reduziertes Dichlorfluorescein-Dichlorfluorescein (DCFH-DCF) ist.

7. Verfahren nach einem der Ansprüche 1 bis 6,
**dadurch gekennzeichnet, dass** die photochemische und kolorimetrische Reaktion bei neutralem pH oder in der Nähe des biologischen pH in einem thermisch stabilisierten Behälter durchgeführt wird.

8. Verfahren nach einem der Ansprüche 1 bis 7,
**dadurch gekennzeichnet, dass** die zu testende biologische Probe eines menschlichen oder tierischen Organismus ein biologisches Fluid wie Serum, Plasma oder ein Gewebeextrakt in einer Lösung ist.

9. Verfahren nach einem der Ansprüche 1 bis 8,
**dadurch gekennzeichnet, dass** für jedes Gemisch die Menge der produzierten chromogenen oder fluoreszierenden Substanz in Form der Intensität des Licht- oder Fluoreszenzsignals der Substanz während eines Zeitraums gemessen wird, der mindestens gleich 45 Minuten, vorzugsweise zwischen 50 und 90 Minuten inklusive, ist.

## Claims

1. A method of measuring the total antioxidant status (TAS) of a biological sample from a human or animal organism, i.e. its ability to withstand reactive oxygen species (ROSs), said method being **characterized in that** it comprises at least the following steps:
· putting the biological sample for testing into contact with a photosensitive agent in a liquid medium to form a mixture for testing;
· subjecting said mixture for testing to a dose of light irradiation at a wavelength that is absorbed by the photosensitive agent in order to give rise, by a photochemical reaction at least between the light and the photosensitive agent, to at least the production of singlet oxygen suitable for co-operating with said sample to form reactive oxygen species (ROSs);
· adding, after irradiation, a compound that reacts colorimetrically in the presence of reactive oxygen species (ROSs) to form a chromogen or fluorescent substance;
· measuring the quantity of chromogen or fluorescent substance produced over time in order to determine the ability of said sample to withstand reactive oxygen species (ROSs) by inhibiting them, a low level of chromogen or fluorescent substance production corresponding to said sample having a high ability to withstand reactive oxygen species (ROSs);
· subjecting at least one reference mixture formed by mixing a reference biological sample from a presumed healthy organism with a photosensitive agent to the same photochemical, colorimetric, and measurement reactions as the mixture for testing; and
· comparing the measurement results on the mixture for testing with the measurement results obtained on said at least one reference mixture.

2. A method according to claim 1, **characterized in that** it comprises, during the measurement step, measuring over time, for each mixture, the quantity of chromogen or fluorescent substance produced in the form of the intensity of the light or fluorescent signal from said substance, said measurement step being followed by a step of processing said measurements, during which, for each mixture, the curve is established for the intensity of the light signal from the chromogen substance as a function of time, and then the area under the curve is calculated and the ratio is determined between the areas of the mixture for testing and of the reference mixture.

3. A method according to any one of claim 1 and claim 2, **characterized in that** prior to putting the sample for testing and the reference sample into contact with the photosensitive agent in order to receive light irradiation, the absorbances of said sample for testing and of the reference sample are measured (to be confirmed).

4. A method according to any one of claims 1 to 3, **characterized in that** a photosensitive agent is used that produces a majority of singlet oxygen ¹O₂, said agent preferably being selected from the group formed by rose bengal and tetra (4 sulfonato-phenyl) porphyrin (TPPS).

5. A method according to any one of claims 1 to 4, **characterized in that** a photosensitive agent is selected that, in the absence of light, does not react with the biological sample for testing, and **in that** said photosensitive agent is irradiated at a selected wavelength corresponding to the spectrum absorption maximum of the photosensitive agent.

6. A method according to any one of claims 1 to 5, **characterized in that** the compound that reacts to form a chromogen substance is the reduced dichlorofluoresceine-dichlorofluoresceine system (DCFH-DCF).

7. A method according to any one of claims 1 to 6, **characterized in that** the photochemical and colorimetric reactions are performed at a pH that is neutral or close to biological pH in a thermostated enclosure.

8. A method according to any one of claims 1 to 7, **characterized in that** the biological sample for testing from a human or animal organism is a biological fluid such as serum, plasma, or a tissue extract in solution.

9. A method according to any one of claims 1 to 8, **characterized in that** for each mixture, the quantity of chromogen or fluorescent substance produced is measured in the form of the intensity of the light or fluorescent signal from said sample over a period of time of not less than 45 min, preferably lying in the range 50 min to 90 min.
